# EUROPEAN PATENT SPECIFICATION

(11) **EP 0 190 014 B1**
(45) Date of publication and mention of the grant of the patent: **16.03.1994**
(21) Application number: 86300457.8
(22) Date of filing: 23.01.1986
(51) Int. Cl.: A61B 1/26

(54) **Medical instruments and illuminating attachments**
Medizinisches Instrument mit zugehöriger Leuchte
Instrument médical et dispositif d'illumination associé

(30) Priority: 23.01.1985 US 694092
(43) Date of publication of application: 06.08.1986
(73) Proprietor: The Trylon Corporation, Harbor City California 90710 (US)
(72) Inventor: Lonky, Neal Marc, Torrance California 90503 (US)
(74) Representative: Thomson, Paul Anthony

(56) References cited:
- US-A- 3 246 646
- US-A- 3 463 915
- US-A- 3 716 047
- US-A- 4 184 193

## Description

This invention relates to an endoscopic device comprising dilator members and including a means to illuminate the body cavity being examined.

The problem of illuminating a body cavity, such as the throat, the vaginal cavity and the rectal cavity, by means of an endoscope has been one that has faced the medical profession for years.

Various kinds of medical apparatus have been proposed for use by physicians in the past to illuminate the interior of different body cavities undergoing inspection and examination. For example, there have been illumination devices provided for examination of the ears, the rectal cavity, the bladder, and the urinary tract. However, such devices often pose problems related to sterilisation prior to use and disposability following use. Further, these devices have not been generally used by the medical profession for vaginal examination and are, in fact, not readily adaptable for such.

The illumination devices which have been proposed in conjunction with specula for vaginal examination have been generally unsatisfactory for a variety of reasons. In many cases they obstruct the vision of the deep internal parts of the vaginal cavity, fail to satisfactorily illuminate the deep interior of the cavity, and, in almost all cases, the proposed device requires sterilisation with the speculum or is designed to come into contact with the body cavity so that it must be sterilised with each use. Finally, most, if not all, known illumination devices utilise an electric light source to provide the illumination needed, thereby subjecting the patient to the possibility of electric shock and the heat of an incandescent light source in a sensitive area.

U.S. Patent No. 4,184,193 relates to a lantern including a chemiluminescent lightstick.

U.S. Patent No. 3,716,047 describes a disposable light-conductive speculum comprising a pair of dilatory blade members and a fiber optic light source, the fiber optic light source being located in one of said dilator blade members and the power for such source being contained within the handle or leg portion of the dilator blade member associated therewith.

As is well-known, an endoscope is ordinarily an instrument, including a light source, for illuminating and thus permitting examination of, the interior of a body cavity. As is also well-known, a speculum is a device for dilating and maintaining open a body cavity to permit examination thereof. A speculum equipped with the medical examination light of the invention, may be considered to be an endoscope, and is so considered herein. The term "endoscopic device", as used herein, is intended to cover both endoscopes provided with dilator members and specula provided with the medical examination light according to claim 1.

It is an object of the invention to provide an endoscopic device adapted to satisfactorily illuminate the body cavity being examined which is simple in construction and, which does not introduce any electrical current into the body cavity or surrounding areas being examined.

It is a preferred object of the invention to provide an endoscopic device which can be made, although not necessarily, of sterilisable material, which may be sterilised after removal of the illuminating means, without causing problems in the sterilising step. Alternatively, the entire device may be made disposable.

According to the present invention there is provided an endoscopic device including an examination illuminating light source for illuminating a body cavity, said endoscopic device comprising:
a) an endoscopic body adapted to receive a light source and comprising a plurality of dilator members, each dilator member extending from a handle or leg portion;
(b) a chemiluminescent light source provided with a light transmitting body adapted to fit adjacent a contoured surface of said endoscopic body, said endoscopic body comprising attachment means associated either with at least one of said dilator members or with said light source in such a way that said chemiluminescent light source is removably secured to said at least one dilator member such as to direct its light between said dilator members in their extended direction.

One aspect of the invention, in an exemplary embodiment, is directed to an endoscopic instrument provided with dilator members and to a medical examination light comprising a chemiluminescent light source is attached. The chemiluminescent light source comprises an hermetically sealed light transmitting tube with at least two compartments separated by a breakable wall, with a chemiluminescent component in each of the compartments. The chemiluminescent light source is attached to a portion of the endoscope by means of an adhesive backing on the light transmitting tube. Following examination, the chemiluminescent light source is readily removed from the endoscope. As indicated above, a speculum to which the medical examination light in attached is considered herein to be an endoscopic instrument.

Another, more specific, aspect of the invention, in an exemplary embodiment, is directed to a vaginal speculum comprising cooperating upper and lower dilator blades operatively connected in a selectively adjustable manner and at least one rib-like member on the surface of at least one of the dilator blades intermediate the dilator blades, having a channel adapted to receive and retain therein, in a releasable, snap-fit, the medical examination light comprising a chemiluminescent light source having an hermetically sealed flexible light transmitting tube with at least two compartments separated by a breakable wall, with a chemiluminescent component in each of the compartments. The tube is adapted to be received and retained in a releasable, snap-fit, in the channel in the rib-like member and to direct its light intermediate the upper and lower dilator blades in their extended position.

These and other objects of the invention will become more apparent from the hereinafter following commentary taken in conjunction with the following figures of drawings.

### BRIEF DESCRIPTION OF THE DRAWINGS

Figure 1 is a side elevational view of the speculum according to the invention showing the illuminating light source in phantom line;
Figure 2 is a top plan view taken along the line 2-2 of Figure 1;
Figure 3 is a view taken along the line 3-3 of Figure 1;
Figure 4 is a cross-sectional view taken along the line 4-4 of Figure 2 showing the illuminating light source;
Figure 5 is a cross-sectional view taken along the line 4-4 of Figure 2 showing the illuminating light source activated;
Figure 6 is a cross-sectional view taken along the line 6-6 of Figure 2;
Figure 7 is a cross-sectional view of an alternate embodiment of the speculum according to the invention taken along the line 6-6 of Figure 2;
Figure 8 is a side elevational view of a medical examination light device of the invention in place on a speculum;
Figure 9 is a view taken along the line 9-9 of Figure 8;
Figure 10̸ is a view taken along the line 10̸-10̸ of Figure 8;
Figure 11 is a view taken along the line 11-11 of Figure 10̸;
Figure 12 is a partial, side elevational view of an alternate embodiment of the speculum according to the invention, showing the illuminating light source in phantom line;
Figure 13 is a view taken along the line 13-13 of Figure 12;
Figure 14 is a view taken along the line 14-14 of Figure 12;
Figure 15 is a view taken along the line 15-15 of Figure 14;
Figure 16 is a partial, cross-sectional view of an alternate embodiment of the speculum according to the invention;
Figure 17 is a view taken along the line 17-17 of Figure 16;
Figure 18 is a view taken along the line 18-18 of Figure 16;
Figure 19 is a cross-sectional view taken along the line 19-19 of Figure 17;
Figure 20̸ is a view similar to Figure 19 illustrating the illuminating light source in a activated state;
Figure 21 is a cross-sectional view of the medical examination light according to the invention, prior to activation;
Figure 22 is a cross-sectional view taken along the line 22-22 of Figure 21;
Figure 23 is a view similar to a portion of Figure 21 showing an alternative embodiment of the plunger portion of the medical examination light;
Figure 24 is a view similar to that of Figure 21 showing the attachment of the medical examination light to an endoscopic instrument with the plunder depicted in the activated position; and
Figure 25 is a side view taken along the line 25-25 of Figure 24.

### DETAILED DESCRIPTION OF THE BEST EMBODIMENTS CONTEMPLATED

Much of the detailed description which follows relates specifically to a vaginal speculum.

Referring to the Figures of drawings wherein like numbers of reference designate like elements throughout, there is illustrated a speculum according to the invention. The instrument consists of a handle 2 connected to a lower dilator blade 4, and upper dilator blade 6 having a second handle 8. Handle 8 is operatively connected to handle 2 in a slidably adjustable manner by means of slot 10̸ in handle 8 which engages around pivot pin 12 on handle 2. The upper dilator blade of the speculum is provided with extending locking tab 14, by means of which the physician can hold in position the remote ends of the two dilator blades separated within the vaginal cavity as the blades are pivoted with respect to one another at pivot pin 12. A plurality of rib-like members 22 are located on the interior surface of upper dilator blade 6 intermediate the two dilator blades and have a channel 24 therein adapted to receive and retain therein, in a releasable, snap-fit, a medical examination light 26.

Figures 6 and 7 illustrate two alternate channels which are capable of receiving and retaining, in a releasable, snap-fit, medical examination light 26 therein.

Medical examination light 26 produces light by chemiluminescent means and has an hermetically sealed, flexible light transmitting tub 28 with at least two compartments 30̸ and 32 separated by a breakable wall 34 with a chemiluminescent component in each compartment. In one compartment in the fluorescer component and in another is the activator component. Mixing of the components produces the chemiluminescent effect, as is well-known. Tube 28 has a cross-sectional shape adapted to be received and retained in channel 24 in a releasable, snap-fitting engagement. Once in position and activated as described below, medical examination light 26 directs its light intermediate the two dilator blades in their extended position to illuminate the cavity for the examining physician.

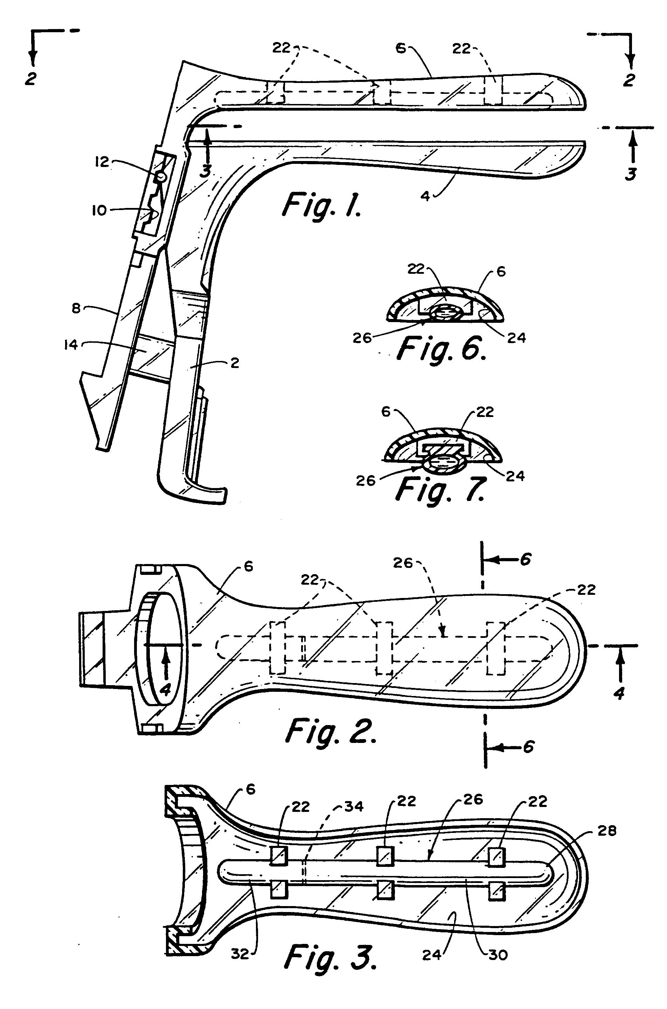

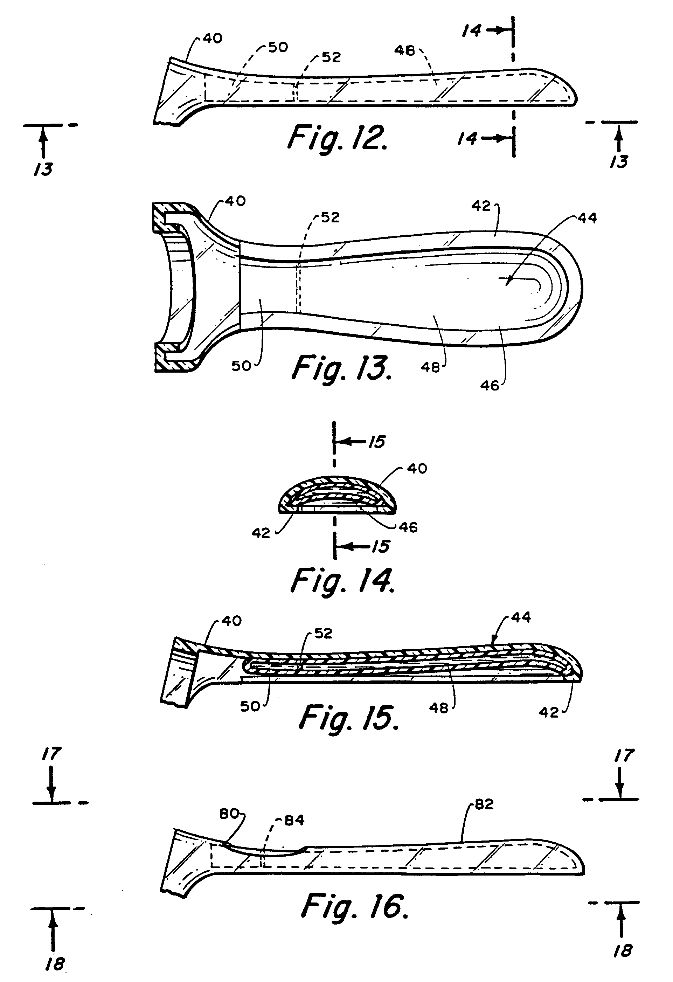

In the embodiment shown in Figures 12-15, only those structural features which differ from those described in Figures 1-5 are shown. Upper dilator blade 40̸ has a perimetric flange 42 along a majority of its lower periphery adapted to receive and retain a medical examination light 44 in a frictional, slip-in fitting engagement adjacent the inner surface of upper dilator blade 40̸ and intermediate the two dilator blades in their extended position.

Medical examination light 44 produces light by chemiluminescent means and has an hermetically sealed, flexible light transmitting bladder member 46 with at least two compartments 48 and 50̸ separated by a breakable well 52 with a chemiluminescent component in each compartment. Bladder member 46 has a shape conformable to the inner surface of upper dilator blade 40̸ so as to be received and retained in a frictional, slip-fitting engagement thereto. More specifically, medical examination light 44 is held in position by having the lower perimetric boundary of its bladder member abut the perimetric flange of the upper dilator blade, thereby preventing the bladder member from moving downward.

In use, the medical examination light will be installed by sliding it into the dilator blade from the rearward position of the dilator blade until it is held in position by the perimetric flange of the dilator blade. To remove the medical examination light, it is pulled toward the rear of the dilator blade once it is no longer needed.

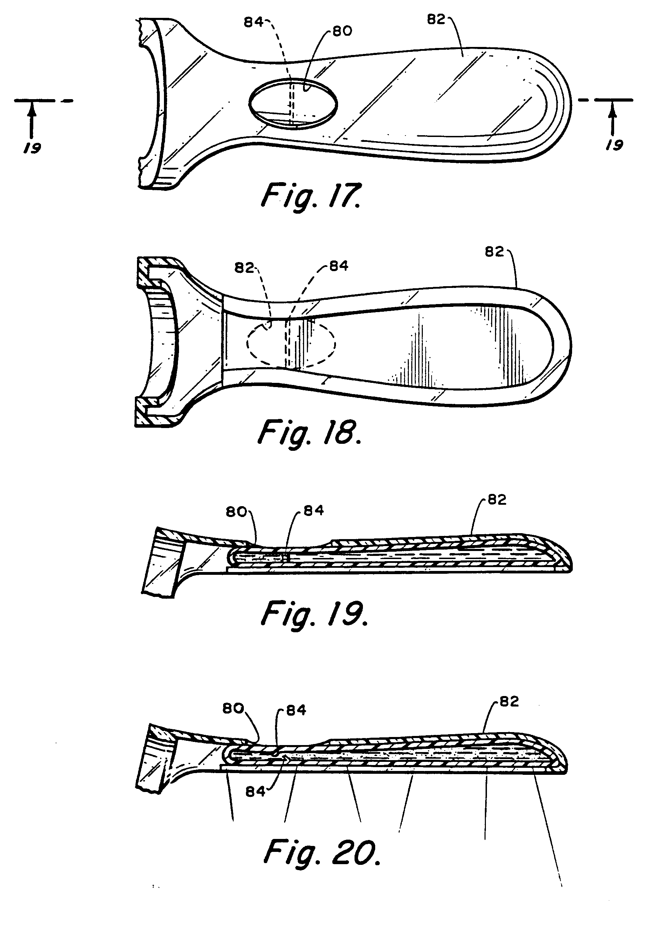

Figures 16-20̸ illustrate an alternate embodiment of the device described immediately above, wherein is added an aperture 80̸ in the extending portion of the dilator blade 82 adopted to permit pressure to be applied, as by a thumb or the like, to rupture the breakable wall 84 and activate the chemical reaction producing chemiluminescence.

The embodiment illustrated in Figures 8-11 shows a partial view of an upper dilator blade 60̸ having the medical examination light removably secured thereto.

The medical examination light 62 has an hermetically sealed flexible light transmitting bladder member 64 with at least two compartments 64 and 68 separated by a breakable wall 70̸ with a chemiluminescent component in each compartment.

Light producing bladder member 64 is attached to the inner surface of a open-ended elastomeric sleeve member 72 generally parallel to the longitudinal axis thereof. Sleeve 72 is generally light transmitting in the area of bladder 64 and is of sufficient size to encircle the extending portion of dilator blade 60̸, receiving and retaining it in a removable, tight friction-fit therein.

To use the medical examination light described herein, the physician first activates the light-producing bladder member, as described elsewhere, and then slips the elastomeric sleeve-member over the extending portion of a dilator blade. The speculum is now ready for the physician's use.

To discard the medical examination light at the conclusion of the examination, the physician need only slip the elastomeric sleeve off the dilator blade by pulling or pushing it toward the remote end of the dilator blade.

In general, to activate the chemiluminescence of the light-containing bladder member, pressure is applied to rupture the breakable wall separating the chemical components necessary to react in order to cause chemiluminescence. The chemiluminescent light source may be activated prior to its attachment to the speculum. This procedure may prove the easiest and preferred method of activating the chemiluminescent light source.

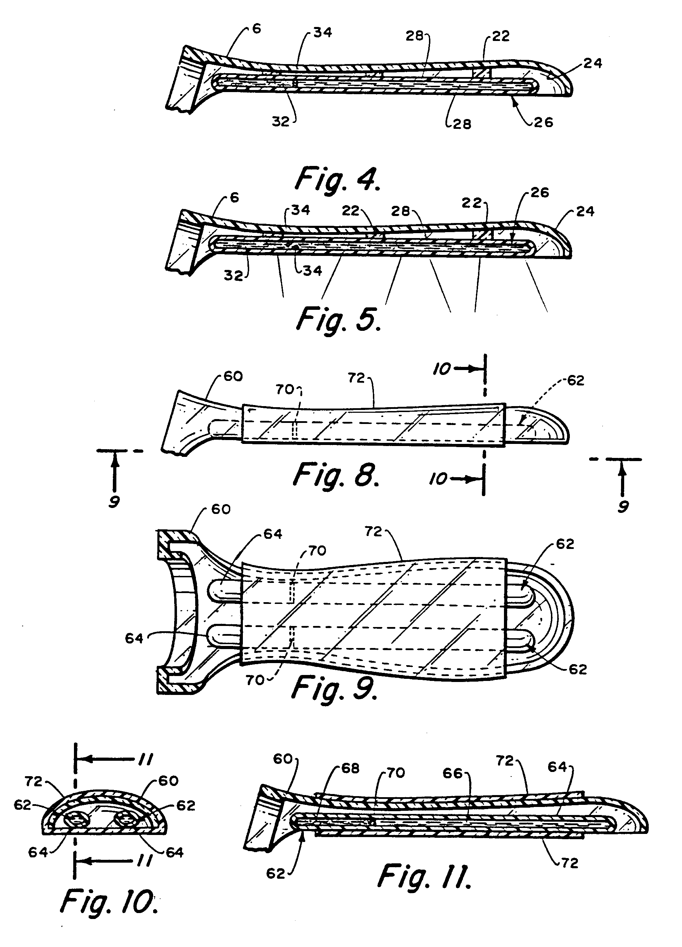

Figure 4 illustrates a chemiluminescent light source attached to a speculum. Figure 5 illustrates the chemical components reacting to cause luminescence.

In alternate embodiments, the rib members may have more than one channel to receive and retain therein, in a releasable snap-fit, more than one chemiluminescent light source as illustrated in Figure 9.

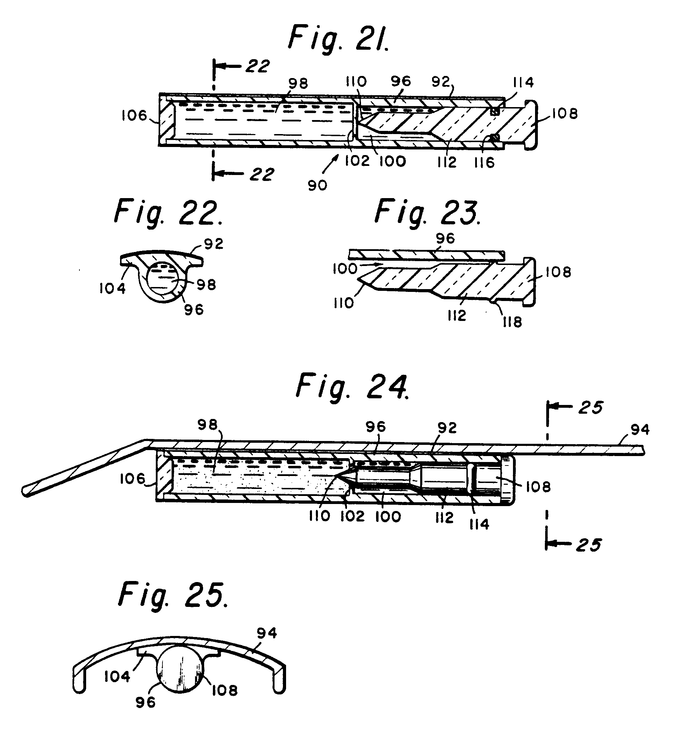

Yet another embodiment of the medical examination light is depicted in Figures 21-25. In this embodiment, the medical examination light 90̸ is provided with an adhesive backing 92, which permits its attachment to an endoscopic instrument (endoscope or speculum) 94 provided with dilator members. The adhesive backing is protected by a layer (not shown) which is peeled off the adhesive when attachment of the medical examination light to the endoscopic instrument is desired.

As described earlier, medical examination light 90̸ produces light by chemiluminescent means and has an hermetically sealed, light transmitting tube 96 with at least two compartments 98 and 10̸0̸ separated by a breakable wall 10̸2 with a chemiluminescent component in each compartment. Tube 96 has a cross-sectional shape adapted to by received the endoscopic device 94 by means of adhesive 92. As shown in Figures 22 and 25, the tube 96 is provided with a flange or rib 10̸4 having a curvilinear shape substantially identical to that of the endoscopic instrument 94 to which it is attached.

Activation to produce the chemiluminescent effect is accomplished by depressing plunger 10̸8 against wall 10̸2 until rupture thereof by point 110̸ of the plunger is achieved, as shown in Figure 24. The two chemiluminescent components in compartments 98 and 10̸0̸ react to produce the desired effect. In operation, the physician would depress the plunger to rupture the wall, optionally shake the tube to speed the chemical reaction, and attach the tube to the endoscopic device by peeling off the adhesive protective layer and securing the "lighted" device to the endoscopic instrument by means of the adhesive.

Hermetic sealing of the compartments 98 and 10̸0̸ is conveniently achieved by providing one of the compartments with an end cap 10̸6 and another of the compartments with the plunger 10̸8.

As shown in Figure 21, shaft 112 of the plunger 10̸8 may be provided with an O-ring seal 114, seated in a slot 116, to provide hermetic sealing of the compartment 10̸0̸ and thus prevent leakage of the chemiluminescent liquid out of the tube 96. Alternatively, as shown in Figure 23, the shaft 112 of the plunger 10̸8 may be provided with a bead 118, integrally formed as part of the plunger, to achieve the same result. Other means may also be suitably employed to provide hermetic sealing and to prevent leakage of the chemiluminescent liquid. Stop means may also be employed to insure retention of plunger 108 in tube 96.

All portions of the medical examination light 90̸ (tube 96, wall 10̸2, end cap 10̸6 and plunger 10̸8) conveniently comprise a plastic material. Examples of such plastic materials include medical grade polystyrene and polypropylene. If polystyrene is employed, which is somewhat rigid, then the wall 10̸2 would comprise a rigid web separating the compartments 98 and 10̸0̸. If a semirigid polypropylene is employed, then the web could have slits or a feather cut therein so as to allow fluid communication between the compartments. In this latter instance, the viscosity of the fluids prevents their intermingling, and hence chemical reaction, until the wall is penentrated by the plunger and the fluids permitted to mix.

In yet another embodiment, at least one of the compartments 98 and 10̸0̸ includes a frangible glass ampoule filled with the appropriate chemiluminescent component. Employing a conformable plastic as the material for the tube 96, then the tube may be squeezed, as between the fingers, to break the ampoule(s) and initiate the chemical reaction. If one of the compartments comprises the glass ampoule, the plunger 10̸8 could be employed to alternatively break the ampoule. Otherwise, if each compartment comprises a glass ampoule, then the plunger could be eliminated altogether and an end cap used in its place.

## Claims

1. An endoscopic device including an examination illuminating light source for illuminating a body cavity, said endoscopic device comprising:
a) an endoscopic body (2,4,6,8; 2,4,40,8; 2,4,60,8; 2,4,94,8) adapted to receive a light source and comprising a plurality of dilator members (4,6; 4,40; 4,60; 4,94), each dilator member extending from a handle or leg portion (2,8);
b) a chemiluminescent light source (26,44,62,90) provided with a light transmitting body (28,46,64,96) adapted to fit adjacent a contoured surface of said endoscopic body, said endoscopic body comprising attachment means (22,42,72,92) associated either with at least one of said dilator members (6,40,60,94) or with said light source (26,44,62,90) in such away that said chemiluminescent light source (26,44,62,90) removably secured to said at least one dilator member (6,40,60,94) such as to direct its light between said dilator members (4,6; 4,40; 4,60; 4,94) in their extended direction.

2. An endoscopic device according to claim 1, characterised in that said plurality of dilator members (4,6) includes cooperating upper (6) and lower (4) dilator blades arranged to engage a cavity in a body and to widen said cavity upon the separation of said dilator members (4, 6), said device preferably including a riser means adjustable on said lower dilator member (4) and a pivot (12) on said riser means pivotally carrying said upper dilator blade (6).

3. An endoscopic device according to claim 1 or 2, characterised in that the attachment means (22,42,72,92) comprises means allowing the light source (26) to be snap-fitted between upstanding ribs (22) on the dilator member (6), means allowing the light source (26) to be slidably fitted with flanges (42) on the dilator member (40), a sleeve (72) that can be fitted over an elongate portion of the dilator member (60).

4. An endoscopic device according to claim 1 or 2, characterised in that the attachment means comprises adhesive means (92) applied to said light source (90).

5. An endoscopic device according to claim 1, 2, 3 or 4, characterised in that said light source comprises:
a) a light transmitting body (28,46,64,96), a portion of the outer surface of which is adapted to cooperate with a contoured surface of said endoscopic body, said portion being provided with attachment means (22,42,72,92) for attachment to said endoscopic body adjacent said surface;
b) at least two hermetically sealed compartments (30,32; 48,50; 64,68; 98,100) within said light transmitting body (28,46,64,96) said compartments being separated by a wall (34,52,70,102), each compartment containing a chemiluminescing component capable of producing chemiluminescent light upon the admixture of the components said wall (34,52,70,102) being rupturable to permit admixture of said components.

6. An endoscopic device according to claim 5, characterised in that said means for rupturing said wall (102) comprises a plunger (108), a portion of which extends into one of said compartments (100), said portion of said plunger (l08) being provided with a point) (ll0) for piercing said wall (l02) and sealing means (ll4,ll8) for maintaining hermetic sealing of said compartment (100).

7. An endoscopic device according to claim 6 characterised in that said sealing means comprises an 0-ring (114) seated in a slot (116) formed in said portion of said plunger (108) or a bead (118) formed on the surface of said portion of said plunger (108),

8. An endoscopic device according to claim 5, characterised in that said wall (102) is the wall of a frangible tube or ampoule containing a chemiluminescing component and arranged within a second deformable compartment containing another chemiluminescing component, said tube or ampoule being easily ruptured by pressure on the deformable compartment.

9. Use of endoscopic device for examining a body cavity according to any one of claims 1 to 8.

## Patentansprüche

1. Endoskopisches Gerät mit einer Lichtquelle zur Untersuchungsbeleuchtung für die Ausleuchtung einer Körperhöhle, wobei das endoskopische Gerät aufweist:
a) einen Endoskopkörper (2, 4, 6, 8; 2, 4, 40, 8; 2, 4, 60, 8; 2, 4, 94, 8), der für die Aufnahme einer Lichtquelle eingerichtet ist und eine Zahl von Dilatationsgliedern (4, 6; 4, 40; 4, 60; 4, 94) aufweist, wobei jedes Dilatationsglied von einem Griff- oder Fußteil ( 2,8) ausgeht;
b) eine Chemolumineszenz-Lichtquelle (26, 44, 62, 90), ausgestattet mit einem lichtdurchlässigen Körper (28, 46, 64, 96), die so ausgebildet ist, daß sie zur Anlage an die Oberflächenkontur einer Oberfläche des Endoskopkörpers angepaßt ist, wobei der Endoskopkörper Befestigungsmittel (22, 42, 72, 92) aufweist, die entweder wenigstens einem der Dilatationsglieder (6, 40, 60, 94) oder der Lichtquelle (26, 44, 62, 90) in solcher Weise zugeordnet sind, daß die Chemoluminszenz-Lichtquelle (26, 44, 62, 90) an wenigstens einem Dilatationsglied (6, 40, 60, 94) lösbar befestigt ist, so daß sie ihr Licht zwischen den Dilatationsgliedern (4, 6; 4, 40; 4, 60; 4, 94) entlang in deren Aufweitungsrichtung leitet.

2. Endoskopisches Gerät nach Anspruch 1, dadurch gekennzeichnet, daß die (Viel-)Zahl der Dilatationsglieder (4, 6) zusammenwirkende obere (6) und untere (4) Dilatationsblätter umfaßt, die so angeordnet sind, daß sie in eine Körperhöhle eingreifen und diese Körperhöhle bei Trennung der Dilatationsglieder (4, 6) aufweiten, wobei das Gerät vorzugsweise ein auf dem unteren Dilatationsglied (4) einstellbares Anhebemittel umfaßt, sowie einen Lagerzapfen (12) auf dem Anhebemittel, der das obere Dilatationsblatt (6) lagert und trägt.

3. Endoskopisches Gerät nach Anspruch 1 oder 2, dadurch gekennzeichnet, daß die Halterungsmittel (22, 42, 72, 92) Mittel umfassen, die es erlauben, die Lichtquelle (26) zwischen aufrechten Rippen (22) auf dem Dilatationsglied (6) einrasten zu lassen, Mittel, die der Lichtquelle (26) erlauben, auf einen Bördelrand (42) am Dilatationsglied (40) aufschiebbar befestigt zu werden, (oder) eine Hülse (72), die auf einem länglichen Teil des Dilatationsgliedes (60) passend aufgesetzt werden kann.

4. Endoskopisches Gerät nach Anspruch 1 oder 2, dadurch gekennzeichnet, daß die Befestigungsmittel an der Lichtquelle (90) angebrachte Klebemittel (92) umfassen.

5. Endoskopisches Gerät nach Anspruch 1, 2, 3 oder 4, dadurch gekennzeichnet, daß die Lichtquelle umfaßt:
a) einen lichtdurchlässigen Körper (28, 46, 64, 96), von dem ein Teil der Außenfläche für das Zusammenwirken mit einer Oberflächenkontur des Endoskopkörpers angepaßt ist, wobei an diesem Teil Befestigungsmittel (22, 42, 72, 92) zum Befestigen an dem an diese Oberfläche angrenzenden Endoskopkörper vorgesehen sind;
b) wenigstens zwei hermetisch verschlossene Kammern (30, 32; 48, 50; 64, 68; 98, 100) innerhalb des lichtdurchlässigen Körpers (28, 46, 64, 96), wobei die Kammern durch eine Wand (34, 52, 70, 102) getrennt sind und jede Kammer eine chemolumineszente Komponente enthält, die in der Lage sind, bei Mischung der Komponenten Chemolumineszenzlicht zu erzeugen, und wobei die Wand (34, 52, 70, 102) zerstörbar ist und das Mischen der Komponenten zuzulassen.

6. Endoskopisches Gerät nach Anspruch 5, dadurch gekennzeichnet, daß die Mittel zum Zerbrechen der Wand (102) einen Kolben (108) umfaßt, dessen einer Teil sich in eine der Kammern (100) erstreckt, wobei dieser Teil des Kolbens (108) eine Spitze (110) zum Durchstechen der Wand (102) aufweist, sowie Dichtmittel (114, 118) zur Gewährleistung eines hermetischen Abschlusses der Kammer (100).

7. Endoskopisches Gerät nach Anspruch 6, dadurch gekennzeichnet, daß die Dichtmittel einen O-Ring (114), der in einem in dem Teil des Kolbens (108) gebildeten Schlitz (116) sitzt, oder (alternativ) einen auf der Oberfläche des Kolben-Teils angeformten Wulst (bead) (118) aufweisen.

8. Endoskopisches Gerät nach Anspruch 5, dadurch gekennzeichnet, daß die Wand (102) die Wand eines zerbrechlichen Tubus oder einer Ampulle ist, die eine chemolumineszente Komponente enthält und innerhalb einer zweiten deformierbaren Kammer angeordnet ist, die eine andere chemolumineszente Komponente enthält, wobei der Tubus oder die Ampulle durch Druck auf die deformierbare Kammer leicht zerstörbar ist.

9. Verwendung des endoskopischen Gerätes nach einem der Ansprüche 1 bis 8 zur Untersuchung einer Körperhöhle.

## Revendications

1. Dispositif endoscopique comprenant une source lumineuse d' éclairage d'examen pour éclairer une cavité corporelle, ledit dispositif endoscopique comprenant :
a) un corps endoscopique (2, 4, 6, 8; 2, 4, 40, 8; 2, 4, 60, 8; 2, 4, 94, 8) adapté pour recevoir une source lumineuse et comprenant une pluralité de pièces de dilatation (4, 6; 4, 40; 4, 60; 4, 94), chaque pièce de dilatation s'étendant à partir de la poignée ou de la partie du pied (2, 8);
b) une source lumineuse chimioluminescente (26, 44, 62, 90) pourvu d'un corps émettant de la lumière (28, 46, 64, 96) adapté pour être ajusté de façon adjacente à une surface adaptée au corps humain dudit corps endoscopique, ledit corps endoscopique comprenant un dispositif de fixation (22, 42, 72, 92) associé soit au moins à une desdites pièces de dilatation (6, 40, 60, 94) soit avec ladite source lumineuse (26, 44, 62, 90) d'une manière telle que ladite source chimioluminescente (26, 44, 62, 90) fixée de façon détachable au moins une dite pièce de dilatation (6, 40, 60, 94) puisse diriger sa lumière entre lesdites pièces de dilatation (4, 6; 4, 40; 4, 60; 4, 94) dans leur direction allongée.

2. Dispositif endoscopique selon la revendication 1, caractérisé en ce que ladite pluralité de pièces de dilatation (4, 6) comprend des lames de dilatation supérieures (6) et inférieures (4) coopérant entre elles disposées pour être en contact avec une cavité d'un corps et pour élargir ladite cavité lors de la séparation desdites pièces de dilatation (4, 6), ledit dispositif comprenant de préférence un dispositif montant ajustable sur ladite pièce de dilatation inférieure (4) et un pivot (12) sur ledit dispositif montant portant de façon pivotante ladite lame de dilatation supérieure (6).

3. Dispositif endoscopique selon la revendication 1 ou 2, caractérisé en ce que ledit dispositif de fixation (22, 42, 72, 92) comprend un dispositif permettant à la source lumineuse (26) d'être encliquetée entre des rebords faisant saillie (22) sur la pièce de dilatation (6), un moyen permettant à la source lumineuse (26) d'être fixée de façon coulissante avec des brides (42) sur la pièce de dilatation (40), un manchon (72) qui peut être fixé sur une partie allongée de la pièce de dilatation (60).

4. Dispositif endoscopique selon la revendication 1 ou 2, caractérisé en ce que ledit dispositif de fixation comprend un moyen adhésif (92) appliqué à ladite source lumineuse (90).

5. Dispositif endoscopique selon la revendication 1, 2, 3 ou 4 caractérisé en ce que ladite source lumineuse comprend :
a) un corps émettant de la lumière (28, 46, 64, 96), dont une partie de la surface externe est adaptée pour coopérer avec la surface adaptée au corps humain dudit corps endoscopique, ladite partie étant pourvue d'un dispositif de fixation (22, 42, 72, 92) pour fixer audit corps endoscopique ladite surface adjacente;
b) au moins deux compartiments fermés hermétiquement (30, 32; 48, 50; 64, 68; 98, 100) dans ledit corps émettant de la lumière (28, 46, 64, 96) ledit compartiment étant séparé par une paroi (34, 52, 70, 102), chaque compartiment comprenant un composant chimioluminescent capable de produire une lumière chimioluminescente lors du mélange des composants, ladite paroi (34, 52, 70, 102) étant cassable pour permettre le mélange desdits composants.

6. Dispositif endoscopique selon la revendication 5, caractérisé en ce que ledit dispositif pour casser ladite paroi (102) comprend un piston (108), dont une partie s'étend dans un desdits compartiments (100), ladite partie dudit piston (108) étant pourvue d'une pointe (110) pour percer ladite paroi (102) et un dispositif d'étanchéité (114, 118) pour maintenir l'étanchéité hermétique dudit compartiment (100).

7. Dispositif endoscopique selon la revendication 6, caractérisé en ce que ledit dispositif d'étanchéité comprend un joint torique (114) placé dans une fente (116) formée dans ladite partie dudit piston (108) ou un bourrelet (118) formé sur la surface de ladite partie dudit piston (108).

8. Dispositif endoscopique selon la revendication 5, caractérisé en ce que ladite paroi (102) est la paroi d'un tube ou une ampoule cassable contenant un composant chimioluminescent et disposée dans un second compartiment déformable contenant un autre composant chimioluminescent, ledit tube ou ladite ampoule étant facilement cassé par pression sur le compartiment déformable.

9. Utilisation d'un dispositif endoscopique pour examiner une cavité corporelle selon l'une quelconque des revendications 1 à 8.
